# EUROPÄISCHE PATENTSCHRIFT

(11) **EP 2 648 773 B1**
(45) Veröffentlichungstag und Bekanntmachung des Hinweises auf die Patenterteilung: **14.01.2015**
(21) Anmeldenummer: 11788386.8
(22) Anmeldetag: 28.11.2011
(51) Int. Cl.: A61L 27/30, A61L 27/06, A61L 27/54, C09D 5/14

(54) **ANTIBAKTERIELLE BESCHICHTUNG FÜR EIN IMPLANTAT UND VERFAHREN ZUR HERSTELLUNG DIESER BESCHICHTUNG**
ANTIBACTERIAL COATING FOR AN IMPLANT AND METHOD FOR PRODUCING SAID COATING
REVÊTEMENT ANTIBACTÉRIEN POUR UN IMPLANT ET PROCÉDÉ DE PRODUCTION DE CE REVÊTEMENT

(30) Priorität: 10.12.2010 DE 102010054046
(43) Veröffentlichungstag der Anmeldung: 16.10.2013
(73) Patentinhaber: DOT GmbH, 18059 Rostock (DE)
(72) Erfinder: NEUMANN, Hans-Georg, 18146 Rostock (DE); PRINZ, Cornelia, 18337 Marlow (DE); LEMBKE, Ulrich, 18147 Rostock (DE)
(74) Vertreter: Prinz & Partner mbB
(86) Internationale Anmeldenummer: PCT/EP2011/005963
(87) Internationale Veröffentlichungsnummer: WO 2012/076124

(56) Entgegenhaltungen:
- US-A1- 2009 198 343
- EWALD ANDREA ET AL: "Antimicrobial titanium/silver PVD coatings on titanium", BIOMEDICAL ENGINEERING ONLINE, BIOMED CENTRAL LTD, LONDON, GB, Bd. 5, Nr. 1, 24. März 2006 (2006-03-24), Seite 22, XP021018047, ISSN: 1475-925X, DOI: 10.1186/1475-925X-5-22
- HEIDENAU F ET AL: "A novel antibacterial titania coating: Metal ion toxicity and in vitro surface colonization", JOURNAL OF MATERIALS SCIENCE: MATERIALS IN MEDICINE, KLUWER ACADEMIC PUBLISHERS, BO, Bd. 16, Nr. 10, 1. Oktober 2005 (2005-10-01), Seiten 883-888, XP019212089, ISSN: 1573-4838, DOI: 10.1007/S10856-005-4422-3

## Beschreibung

Die Erfindung betrifft eine antibakterielle Beschichtung für ein Implantat sowie ein Verfahren zur Herstellung dieser Beschichtung.

Es ist allgemein bekannt, dass Silber eine hohe antibakterielle Wirksamkeit hat. Es ist im Stand der Technik auch bereits beschrieben, Silber in einer Beschichtung von Implantaten zu verwenden. Beispiele finden sich in der WO 2010/106164 A1 und der EP 1 632 198 B1. Eine solche Beschichtung bietet Vorteile, wenn das Implantat während der Operation oder in der Operationswunde mit Bakterien in Berührung kommt, beispielsweise wenn während der Operation ungewollt Bakterien in die Wunde gelangen oder in der Wunde, auf Grund einer vorhandenen Infektion, schon Bakterien vorhanden sind.

Aus der Literatur ist aber auch bekannt, dass Silberionen ebenso eine zytotoxische Wirkung besitzen. Diese Wirkung setzt schon bei Konzentrationen von größer als 380 µg/l ein (siehe bespielsweise Heidenau, F., Mittelmeier W., Detsch R., Haenle M., Stenzel F., Zeigler G., Gollwitzer H., 2005: "A novel antibacterial titania coating: Metall ion toxicity and in vitro surface colonization", J. Materials Science, Materials in Medicine 16, 1-6").

Die bereits bei derart geringen Konzentrationen beobachtete Zelltoxizität von Silberionen korrespondiert damit, dass Silber im Stoffwechsel der Zelle keine Rolle spielt; es gehört nicht zu den so genannten essentiellen Spurenelementen im menschlichen Organismus. Die Einnahme von niedrigen Dosen Silber wie auch ein über längere Zeit stattfindendes Release aus Silberoberflächen kann zu dauerhaften Zellschädigungen führen. Die WHO empfiehlt, pro Tag nicht mehr als 180 µg Silber zu konsumieren (siehe beispielsweise Gibbins, B., Warner, L., 2005: "The Role of Antimicrobial Silver Nanotechnology", MDDI, http://www.devicelink.com/mddi/archive/05/08/005.html, 05.02.08).

Es ist ebenfalls bekannt, dass Kupfer eine antibakterielle Wirkung hat. Diese ist jedoch geringer als die antibakterielle Wirkung von Silber. Im Gegensatz zu Silber rufen Kupferionen in maßvollen Konzentrationen keine Zellschädigungen hervor (siehe die o.g. Veröffentlichung von Heidenau et al.); sie sind hingegen für den Stoffwechsel der Zellen von essentieller Bedeutung und in den Körpermedien mit Konzentrationen von 11 - 24 µmol/l vorhanden (siehe beispielsweise Katalog des Inst. f. Labordiagnostik Klinikum Süd, Rostock, 2005).

Dementsprechend sind im Stand der Technik auch bereits antibakterielle Beschichtungen beschrieben, die Kupfer verwenden. Ein Beispiel findet sich in der US 5,958,440, bei der eine antibakterielle Beschichtung mit u.a. Kupfer mittels eines PVD-Verfahrens (physical vapour deposition; physikalische Gasphasenabscheidung) aufgebracht wird. Allerdings ergeben sich dort nur Schichten mit einer begrenzten Härte, die für einige Anwendungen nicht geeignet sind.

Aus der US 2009/0198343 A1 ist eine antibakterielle PVD-Beschichtung bekannt, die auf ein Implantat aufgebracht werden kann und Kupfer sowie Silber enthält.

In Ewald et al. "Antimicrobial titanium/silver PVD coatings on titanium" ist eine Titan-Silber-Beschichtung für ein Implantat aus Titan beschrieben.

Aus Heidenau et al. im Journal of Materials Science ist die Wirkung von Kupfer- und Silberionen bezüglich ihrer antibakteriellen und der zellschädigenden Wirkung bekannt.

Aufgabe der Erfindung ist es, Oberflächenschichten aufzuzeigen, die die vorhandenen Vorzüge der PVD-Schichten wie gute Haftfestigkeit und große Härte mit einer antibakteriellen Wirkung der Schicht kombinieren, sowie ein Verfahren zur effektiven Herstellung derartiger Oberflächenschichten.

Zur Lösung dieser Aufgabe ist erfindungsgemäß eine antibakterielle Beschichtung für ein Implantat vorgesehen, die dadurch gekennzeichnet ist, dass sie mindestens eine Schicht aus Kupfer-Titan-Nitrid enthält. Die Erfindung beruht auf der Erkenntnis, dass sich durch Hinzufügen von Kupfer zur Beschichtung eine antibakterielle Wirkung erzielen lässt, ohne dass dadurch die mechanischen Eigenschaften der Beschichtung wesentlich nachteilig beeinflusst werden, insbesondere die Härte, und ohne dass sich dadurch der Herstellungsaufwand erhöht. Dabei wird berücksichtigt, dass bereits vergleichsweise geringe Mengen an Kupfer eine ausreichende antibakterielle Wirkung haben, obwohl Kupfer an sich eine geringere antibakterielle Wirkung hat als Silber. Dem gegenüber beginnt die zelltoxische Wirkung von Silber bei deutlich niedrigeren Konzentrationen als bei Kupfer. Die Schicht aus Kupfer-Titan-Nitrid knüpft an die bereits bekannten und bewährten Titan-Nitrid-Beschichtungen an, die insbesondere bei Implantaten verwendet werden. Durch den zusätzlichen Anteil an Kupfer lässt sich erfindungsgemäß eine antibakterielle Wirkung erzielen, ohne dass die anderen Eigenschaften und die sich daraus ergebenden Vorteile dieser Beschichtung beeinträchtigt werden.

Die antibakterielle Wirkung ermöglicht, die Aktivität von Bakterien, die während des Einsetzens des Implantats in den Körper ungewollt auf das Implantat oder in die Wunde gelangen oder aufgrund einer Infektion bereits im Körper vorhanden sind, erheblich zu reduzieren. Wenn die Aktivität von eventuell vorhandenen Bakterien reduziert wird, kann sich das neu bildende Knochengewebe besser mit dem Implantat verbinden. Außerdem müssen nach der Operation weniger Medikamente gegen Infektionen gegeben werden, was sich insgesamt positiv auf den Heilungsprozess nach der Implantation auswirkt.

Unter dem Begriff "Implantat" wird hier jedes Bauteil verstanden, das dafür vorgesehen ist, über einen längeren Zeitraum im Körper eines Menschen oder eines Tieres zu verbleiben, z.B. als Knochennagel, Teil eines künstlichen Gelenks, Knochenersatz, künstliches Organ, etc.

Vorzugsweise ist vorgesehen, dass die Beschichtung neben Kupfer und Titan mindestens einen der folgenden Bestandteile enthält: Zr, Nb, Ta, Cr, Mo, W, Si, Al. Mit diesen Bestandteilen lässt sich eine sehr hohe Härte der Beschichtung auch bei der Anwesenheit von Kuper erzielen. Besonders bevorzugt werden die biokompatiblen Bestandteile Ta_und Nb.

Mindestens ein metallischer Bestandteil kann in der Form von Nitriden, Carbiden oder Oxiden gebunden sein. Nitride und Carbide haben den Vorteil einer besonders hohen Härte. Oxide bieten Vorteile hinsichtlich ihrer Oberflächenstruktur. So können auch Schichten mit unterschiedlichen tribologischen Eigenschaften und unterschiedlicher Temperaturbeständigkeit erzeugt werden. Die Möglichkeit, Schichten mit günstigen tribologischen Eigenschaften zu erzeugen, ist insbesondere für Implantate von großer Bedeutung.

Bei mehrlagigen Schichten ist dabei ein Wechsel zwischen reinen Metallschichten und Schichten mit Nitriden, Oxiden und Carbiden möglich, wobei jede einzelne Schicht Kupfer enthalten kann oder nicht, aber erfindungsgemäß mindestens eine Schicht Kupfer derart enthält, dass Kupferionen freigesetzt werden können und die Freisetzung durch darüber liegende Schichten nicht vollständig verhindert, sondern höchstens in einem erwünschten Maße verzögert wird.

Der wählbare Anteil von Kupfer in der erfindungsgemäßen Schicht ist eine Funktion der gewünschten maximalen Kupferkonzentration in der das Implantat umgebenden Körperflüssigkeit, der Geschwindigkeit, mit der Kupfer in der entsprechenden Umgebung abgebaut wird, der Freisetzungsrate von Kupfer aus der erfindungsgemäßen Schicht sowie des Zeitraums, in dem eine Freisetzung von Kupfer erfolgen soll. Dabei sollte eine Konzentration von 200 µmol/l nicht überschritten werden, da ab dieser Konzentration das Kupfer zelltoxisch wirkt.

Vorzugsweise ist vorgesehen, dass die Beschichtung Kupfer in einem solchen Anteil aufweist, dass Kupfer mit einer Rate freigesetzt werden kann, dass sich in einer das Implantat umgebenden Testflüssigkeit, die physiologisch einer Körperflüssigkeit in einer Wunde des Körpers entspricht, eine Kupfer-Konzentration von 90 bis 160 µmol/l ergibt. Da bereits ein deutlich niedrigerer Anteil von Kupfer in der umgebenden Körperflüssigkeit eine ausreichende antibakterielle Wirkung hat, kann die erfindungsgemäße Beschichtung so gestaltet werden, dass der oben genannte Grenzwert deutlich unterschritten wird. Vorzugsweise wird eine Konzentration von 90 bis 160 µmol/l Kupfer in der umgebenden Körperflüssigkeit angestrebt. Gemäß einer Ausführungsform ist vorgesehen, dass die Beschichtung einlagig ist. Auf diese Weise ergibt sich ein geringer Herstellungsaufwand.

Alternativ ist vorgesehen, dass die Beschichtung mehrlagig ist. Dies ermöglicht, unterschiedliche Anforderungen an das Implantat durch das Aufbringen von unterschiedlichen Schichten innerhalb der Beschichtung zu erfüllen.

Mit unterschiedlichen Schichten innerhalb der Beschichtung ist es insbesondere möglich, die Freisetzungsrate der Kupferionen an die Umgebung zu kontrollieren. So kann beispielsweise eine erste Schicht der mehrlagigen Beschichtung den Kupfer-Anteil enthalten und über dieser Schicht eine Verzögerungsschicht aus TiN aufgebracht sein, deren Schichteigenschaften die Freisetzungsrate der Kupferionen an der Schichtoberfläche beeinflussen.

Vorzugsweise liegt die vollständige antibakterielle Beschichtung in einer Stärke von 1 bis 7 µm vor. Besonders bevorzugt ist eine Stärke von 2 bis 4 µm. Es hat sich herausgestellt, dass sich bereits mit einer derart dünnen Beschichtung die gewünschte antibakterielle Wirkung erzielen lässt.

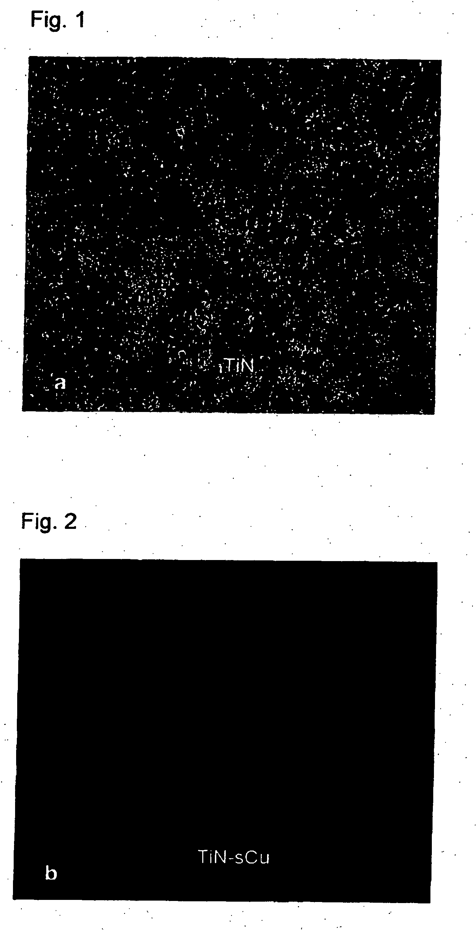

Die Erfindung wird nachfolgend anhand einer Ausführungsform unter Bezugnahme auf die beigefügen Figuren 1 und 2 beschrieben.

Grundsätzlich gilt, dass die erfindungsgemäße antibakterielle Beschichtung des Implantats als dünne metallische und/oder keramische Schicht auf einer oder mehreren Oberflächen des Implantats ausgeführt ist. Diese Schicht enthält die Atome von mindestens 2 Metallen, von denen eines immer das antibakteriell wirkende Kupfer ist. Darüber hinaus können mindestens ein weiteres Metall, aber auch beliebige Kombinationen aus den folgenden Metallen enthalten sein: Ti, Zr, Nb, Ta, Cr, Mo, W, Si, Al. Die erfindungsgemäßen Schichten können darüber hinaus keramische Verbindungen mit Stickstoff, Sauerstoff und Kohlenstoff enthalten. Die erfindungsgemäßen Schichten können überall dort eingesetzt werden, wo heute schon PVD-Schichten der genannten Metalle und keramischen Verbindungen verwendet werden und zusätzlich eine antibakterielle Wirkung der Schicht erwünscht ist.

Zur Herstellung der Beschichtung werden PVD-Verfahren verwendet, wie sie grundsätzlich zum Abscheiden von Schichten mit großer Härte bekannt sind. In Abhängigkeit von den Anforderungen können folgende Vakuum-gestützte Beschichtungsverfahren verwendet werden: Bogenverdampfung, thermische Verdampfung, Hohlkathodenverdampfung, Kathodenzerstäubung (Sputtern) und Ionenplattierung. Die jeweiligen Anforderungen bestimmen auch, ob Mischtargets und/oder reine Targets verwendet werden.

Nachfolgend wird ein beispielhaftes Verfahren zur Beschichtung von Implantaten mit der antibakteriellen Beschichtung beschrieben. Die Implantate werden mit einer Beschichtung versehen, die aus einem Mehrschichtsystem von TiN und Cu in mehreren Unterschichten besteht. Der Anteil an Kupfer in der Beschichtung ist ausreichend hoch, um eine antibakterielle Wirkung zu entfalten. Gleichzeitig ist die Konzentration der im Körper freigesetzten Kupferionen nicht so hoch, dass es zu einer zellschädigenden Wirkung und damit zu einer Beeinträchtigung des Anwachsens des Implantats kommt. Insbesondere soll eine Kupfer-Konzentration vermieden werden, die 200 µmol/l Körperflüssigkeit überschreitet.

Die antibakterielle Wirkung der beispielhaften Beschichtung wurde mittels verschiedener Titanproben nachgewiesen, die mit einer mehrlagigen Beschichtung versehen werden. Die Beschichtung wurde mit einem PVD-Verfahren abgeschieden und aus Titan und Kupfer gebildet. Dabei wurde als Reaktivgas Stickstoff zugeführt. Eine solche Beschichtung eignet sich insbesondere als harte Beschichtung für Implantate.

Die Titanproben wurden mit einer wässrigen Waschlösung in einem Ultraschallbad gewaschen, dann mit destilliertem Wasser gespült und anschließend in einem Trockenschrank getrocknet.

Nach der Trocknung wurden die Proben in der Vakuumkammer der PVD-Anlage auf einem Drehteller platziert. Durch den Drehteller wird eine zweiachsige Rotation der Titanproben herbeigeführt. So kann eine homogene Beschichtung im gerichteten Strahl der Beschichtungsquellen gewährleistet werden.

Die verwendete PVD-Anlage verfügte über zwei Quellen, nämlich eine Bogen-Verdampferquelle und eine Magnetron-Sputterquelle. In der Bogen-Verdampferquelle befand sich ein Titantarget, während in der Magnetron-Sputterquelle ein Kupfertarget verwendet wurde.

Durch Abpumpen der Kammer wurde der Arbeitsdruck kleiner als 0,025 mbar erzeugt. Für die ersten Arbeitsschritte wurde kein Reaktivgas verwendet.

Anschließend wurde die Oberfläche der Titanproben in zwei Arbeitsschritten gereinigt und aktiviert. Zuerst wurden die Proben bei 300 °C für 45 min ausgeheizt. Danach wurden die Proben bei einer Vorspannung von -900 V für 2 min mit Titanionen aus der Bogen-Quelle gesputtert.

In einem weiteren Arbeitsschritt erfolgte für 3 min ebenfalls unter Verwendung der Bogen-Quelle die Aufbringung einer Titan-Haftschicht bei einer Vorspannung der Proben von -150 V und einem Verdampferstrom von 70 A. Beide Werte wurden für die Bogen-Quelle auch in den folgenden Schritten verwendet.

Während der nächsten Arbeitsschritte erfolgte die Einleitung einer definierten Rate des Reaktivgases Stickstoff in die Vakuumkammer.

Als nächste Schicht wurde für 10 min von der Bogen-Quelle Titannitrid (TiN) auf den Proben abgeschieden.

Anschließend wurde die erfindungsgemäße Schicht als mehrlagige Sandwichschicht erzeugt. Beginnend mit Kupfer aus der Magnetron-Sputterquelle (Leistung 500 W) im Wechsel mit Titan aus der Bogen-Quelle wurden so 24 Schichten erzeugt, je 12 Kupferschichten und 12 Titannitridschichten. Die letzte Schicht ist Titannitrid (TiN). Die Beschichtungszeit betrug für jede Titannitrid- und Kupferschicht jeweils 2 min.

Anschließend wurde die Schichtdicke gemessen und die Elementzusammensetzung mit einer EDX-Analyse (Energiedispersive Röntgenspektroskopie) ermittelt.

Die gesamte Schichtdicke betrug im Durchschnitt der Proben 2,13 µm und die Kupferkonzentration liegt im Durchschnitt bei 3,3 µg/mm².

Anschließend wurde die Freisetzung von Kupfer aus der Oberfläche untersucht. Um den Bedingungen im Körper möglichst nahe zu kommen, wurden dazu beschichtete Proben bei 37 °C in PBS-Puffer und Zellkulturmedium gelegt und die freigesetzte Kupfermenge in festgelegten Zeitabständen gemessen. Zur Ermittlung des Kupfergehaltes in den Lösungen wurden die photometrische Bestimmung und die Atomabsorptionsspektroskopie (AAS) verwendet.

Die dabei gemessene kumulative Kupferkonzentration kann der nachfolgenden Tabelle 1 entnommen werden:

| Tage | 0 | 1 | 2 | 3 | 4 | 5 | 6 | 7 | 8 | 9 | 10 |
|---|---|---|---|---|---|---|---|---|---|---|---|
| mg/l | 0 | 13,5 | 15,4 | 16,8 | 26,2 | 28,7 | 32,1 | 32,5 | 32,9 | 33,3 | 34,4 |

Die Untersuchung der Bakterienaktivität wurde mit Bakterienkulturen in PBS-Puffer und in Zellkulturmedium untersucht. Alle Proben und Probengefäße wurden vor dem Beimpfen mit Bakterien im Autoklaven sterilisiert. Alle folgenden Arbeiten wurden unter sterilen Bedingungen durchgeführt. Alle bakteriellen Versuche wurden mit Staphylococcus aureus ATCC 25923 (erhältlich von Firma DOENITZ-PROLAB) durchgeführt.

Da bekannt ist, dass sich Mikroorganismen, die in Biofilmen leben, deutlich von planktonischen Bakterien unterscheiden, wurden beide Arten untersucht. Besonders die Bildung eines Biofilmes ist auf Implantatoberflächen unerwünscht. Die Wirkung der erfindungsgemäßen Schicht wurde für beide Arten untersucht. Dazu wurden die Proben bei 37 °C mit einer festgelegten Menge von Bakterien (1,00E+07 KBE/ml) in die überstehende Lösung gelegt und die Anzahl der Bakterien, bei den planktonischen Bakterien in der Lösung und bei den biofilmbildenden Bakterien auf der Substratoberfläche, zu festgelegten Zeiten bestimmt.

Die dabei gemessenen Werte können der nachfolgenden Tabelle 2 entnommen werden:

| | TiCuN | TiN | PK PBS |
|---|---|---|---|
| 0 h | 9,60E+06 | 9,60E+06 | 9,60E+06 |
| 2 h | 3,50E+04 | 4,10E+05 | 3,07E+05 |
| 4 h | 1,32E+03 | 2,03E+06 | 1,98E+06 |
| 6 h | 6,90E+02 | 1,34E+07 | 6,30E+06 |
| 8 h | 6,00E+03 | 4,55E+06 | 1,29E+06 |
| 24 h | 5,00E+01 | 2,49E+06 | 7,13E+06 |

Es ist zu sehen, dass eine stark antibakterielle Wirkung vorhanden ist, obwohl bei den Proben die Kupfer-Schicht nicht an der Oberfläche der Probe freiliegt, sondern von der letzten Schicht aus Titannitrid (TiN) bedeckt ist.

In der Praxis werden zur Beschichtung des Implantats üblicherweise weniger Schichten verwendet als bei der beschriebenen Probe. Wesentlich ist, dass durch die geeignete Ausführung der Kupfer enthaltenden Schichten (insbesondere hinsichtlich Dicke und Kupfergehalt) in Kombination mit der Abschlussschicht die Freisetzungsrate und Freisetzungsdauer auf vorteilhafte Werte eingestellt werden kann. Die Abschlussschicht kann dabei in Abhängigkeit von den Anforderungen aus TiN, TiO, TiC oder auch nur Ti bestehen. Zusätzlich kann Ta oder Nb beigefügt sein oder auch nur Ta oder Nb verwendet werden.

Alternativ zu einer Beschichtung, die aus mehreren abwechselnden Lagen aus Kupfer und Titannitrid besteht, kann auch eine einlagige Beschichtung aufgebracht werden, die aus TiCuN besteht. Auch auf diese Weise kann eine stark antibakterielle Wirkung erzielt werden, wie der Vergleich der Figuren 1 und 2 zeigt. Die Figuren 1 und 2 zeigen die Oberflächen von Proben, die denselben Bedingungen ausgesetzt waren. Bei Figur 1 handelt es sich um eine Probe aus Titannitrid. Die Besiedlung durch Staphylococcus aureus ist deutlich zu erkennen. Figur 2 zeigt eine mit Titannitrid und Kupfer beschichtete Probe. Die Oberflächen ist nahezu keimfrei.

Eine einlagige Beschichtung kann mit einem Mischtarget hergestellt werden, das aus einer Kupferlegierung mit einem oder mehreren der Metalle Ti, Zr, Nb, Ta, Cr, Mo, W, Si, Al besteht. Der Vorteil dieses Verfahrens ist der geringere Herstellungsaufwand. Dafür bestehen gewisse Einschränkungen, die Freisetzungsrate des Kupfers zu steuern.

## Patentansprüche

1. Antibakterielle Beschichtung für ein Implantat, **dadurch gekennzeichnet, dass** sie mindestens eine Schicht aus Kupfer-Titan-Nitrid enthält.

2. Beschichtung nach Anspruch 1, **dadurch gekennzeichnet, dass** sie mindestens einen der folgenden Bestandteile enthält: Zr, Nb, Ta, Cr, Mo, W, Si, Al.

3. Beschichtung nach Anspruch 2, **dadurch gekennzeichnet, dass** sie die biokompatiblen Metalle Ta und Nb enthält.

4. Beschichtung nach einem der vorhergehenden Ansprüche, **dadurch gekennzeichnet, dass** ein Beschichtungsbestandteil in der Form von Nitriden, Carbiden oder Oxiden gebunden ist.

5. Beschichtung nach einem der vorhergehenden Ansprüche, **dadurch gekennzeichnet, dass** sie aus einer einzigen Schicht besteht.

6. Beschichtung nach einem der Ansprüche 1 bis 4, **dadurch gekennzeichnet, dass** sie aus mehreren Schichten besteht.

7. Beschichtung nach Anspruch 6, **dadurch gekennzeichnet, dass** mehrere Schichten vorgesehen sind, deren Aufbau unerschiedlich ist.

8. Beschichtung nach Anspruch 7, **dadurch gekennzeichnet, dass** eine oder mehrere Schichten der mehrlagigen Beschichtung den Kupfer-Anteil enthalten und über diesen Schichten eine oder mehrere Verzögerungsschichten aufgebracht sind, die keinen Kupfer-Anteil enthalten.

9. Beschichtung nach einem der vorhergehenden Ansprüche, **dadurch gekennzeichnet, dass** sie in einer Stärke von 1 µm bis 7 µm vorliegt, vorzugsweise in eine Stärke von 2 bis 4 µm.

10. Beschichtung nach einem der vorhergehenden Ansprüche, **dadurch gekennzeichnet, dass** sie auf ein Implantat aufgebracht ist.

11. Beschichtung nach Anspruch 10, **dadurch gekennzeichnet, dass** das Implantat aus Titan oder einer Titanlegierung besteht.

12. Beschichtung nach einem der vorhergehenden Ansprüche, **dadurch gekennzeichnet, dass** es sich um eine PVD-Beschichtung handelt.

13. Beschichtung nach einem der vorhergehenden Ansprüche, **dadurch gekennzeichnet, dass** sie Kupfer in einem solchen Anteil aufweist, dass Kupfer mit einer Rate freigesetzt werden kann, dass sich in einer das Implantat umgebenden Körperflüssigkeit eine Kupfer-Konzentration von 90 bis 160 µmol/l ergibt.

## Claims

1. An antibacterial coating for an implant, **characterized in that** the coating contains at least one layer of copper titanium nitride.

2. The coating according to claim 1, **characterized in that** it contains at least one of the following components: Zr, Nb, Ta, Cr, Mo, W, Si, Al.

3. The coating according to claim 2, **characterized in that** it contains the biocompatible metals Ta and Nb.

4. The coating according to any of the preceding claims, **characterized in that** a coating component is bound in the form of nitrides, carbides or oxides.

5. The coating according to any of the preceding claims, **characterized in that** it consists of one single layer.

6. The coating according to any of claims 1 to 4, **characterized in that** it consists of a plurality of layers.

7. The coating according to claim 6, **characterized in that** a plurality of layers are provided which have different structures.

8. The coating according to claim 7, **characterized in that** one or more layers of the multilayer coating contain the copper content, and applied over these layers are one or more retardation layers which do not contain a copper content.

9. The coating according to any of the preceding claims, **characterized in that** it is provided in a thickness of from 1 µm to 7 µm, preferably in a thickness of from 2 to 4 µm.

10. The coating according to any of the preceding claims, **characterized in that** it is applied on an implant.

11. The coating according to claim 10, **characterized in that** the implant consists of titanium or a titanium alloy.

12. The coating according to any of the preceding claims, **characterized in that** it is a PVD coating.

13. The coating according to any of the preceding claims, **characterized in that** it includes copper in such a proportion that copper can be released at a rate such that a copper concentration of from 90 to 160 µml/l is obtained in a body fluid surrounding the implant.

## Revendications

1. Revêtement antibactérien pour un implant, **caractérisé en ce qu'**il contient au moins une couche en cuivre-titane-nitrure.

2. Revêtement selon la revendication 1, **caractérisé en ce qu'**il contient au moins l'un des composants suivants : Zr, Nb, Ta, Cr, Mo, W, Si, Al.

3. Revêtement selon la revendication 2, **caractérisé en ce qu'**il contient les métaux biocompatibles Ta et Nb.

4. Revêtement selon l'une des revendications précédentes, **caractérisé en ce qu'**un composant de revêtement est lié sous forme de nitrures, de carbures ou d'oxydes.

5. Revêtement selon l'une des revendications précédentes, **caractérisé en ce qu'**il est composé d'une seule couche.

6. Revêtement selon l'une des revendications 1 à 4, **caractérisé en ce qu'**il est composé de plusieurs couches.

7. Revêtement selon la revendication 6, **caractérisé en ce qu'**il est prévu plusieurs couches présentant des structures différentes.

8. Revêtement selon la revendication 7, **caractérisé en ce qu'**une ou plusieurs couches du revêtement multicouche contient/contiennent la part de cuivre, et **en ce qu'**une ou plusieurs couche(s) retardatrice(s) ne contenant pas de part de cuivre est/sont appliquée(s) au-dessus de ces couches.

9. Revêtement selon l'une des revendications précédentes, **caractérisé en ce qu'**il est présent avec une épaisseur de 1 µm à 7 µm, de préférence avec une épaisseur de 2 µm à 4 µm.

10. Revêtement selon l'une des revendications précédentes, **caractérisé en ce qu'**il est appliqué sur un implant.

11. Revêtement selon la revendication 10, caractérisé en que l'implant est en titane ou en un alliage de titane.

12. Revêtement selon l'une des revendications précédentes, **caractérisé en ce qu'**il s'agit d'un revêtement PVD.

13. Revêtement selon l'une des revendications précédentes, **caractérisé en ce qu'**il présente du cuivre dans une telle proportion que du cuivre peut être libéré avec un taux tel qu'il en résulte une concentration de cuivre de 90 à 160 µmol/l dans un fluide corporel entourant l'implant.
